# EUROPEAN PATENT SPECIFICATION

(11) **EP 1 095 260 B1**
(45) Date of publication and mention of the grant of the patent: **10.11.2004**
(21) Application number: 99931612.8
(22) Date of filing: 06.07.1999
(51) Int. Cl.: G01N 21/25, G01N 21/03, G01N 33/22

(54) **OPTICAL PROCESS OF DETECTING DYED DIESEL IN NON-DYED DIESEL**
OPTISCHES VERFAHREN ZUR DETEKTION VON FARBMARKIERTEM DIESELKRAFTSTOFF IN UNMARKIERTEM DIESELKRAFTSTOFF
PROCEDE OPTIQUE DE DETECTION DE DIESEL COLORE DANS DU DIESEL NON COLORE

(30) Priority: 07.07.1998 NO 983126
(43) Date of publication of application: 02.05.2001
(73) Proprietor: Miljo-Energi AS, 5058 Bergen (NO)
(72) Inventor: HAMMER, Erling, A., N-5136 Mjolkeräen (NO)
(74) Representative: Wallin, Bo-Göran
(86) International application number: PCT/NO1999/000227
(87) International publication number: WO 2000/002034

(56) References cited:
- EP-A2- 0 304 230
- WO-A1-99/00666
- US-A- 4 810 090
- US-A- 5 172 192
- US-A- 5 696 592
- DATABASE WPI Week 198640, Derwent Publications Ltd., London, GB; AN 1986-263943/40, XP002940749 & SU 1 213 392 A (GORENKOV AF) 23 February 1986
- PATENT ABSTRACTS OF JAPAN vol. 009, no. 167 (P-372) 12 July 1985 & JP 60 044 851 A (SHIMAZU SEISAKUSHO KK) 11 March 1985

## Description

The present invention relates to a process for detecting dyed diesel in undyed diesel.

In several countries, including Norway and Sweden, there is adopted a differentiated duty system for diesel oils, i.e. there is a distinction between duty-levied and duty-free diesel oils. In order to distinguish these from each other dyes plus a tracer have been added to the duty-free diesel, in Norway a red dye, and in Sweden a green dye. By virtue of the price difference between the two diesel variants extensive illegal use of the duty-free diesel has occurred.

There is therefore an expressed wish from the Duty Directorate for a handy measuring instrument which can be guided down into fuel tanks of motor vehicles in order to prove illegal use of such dyed duty-free diesel.

To-day there is employed a complicated chemical method of detection for identifying whether there exist fractions of dyed diesel in non-dyed diesel. This method of detection (based on DIN 51426) is based on a demonstration and a quantitative detection of Solvent Yellow 124 (tracer), which is employed inter alia in Norway as the stable tracer label in dyed diesel. The method is complicated and time-consuming, and employes a series of chemicals, inter alia hydrochloric acid and organic solvents. A standard calibration curve has to be prepared and the method also comprises a chromatography purificating step. This implies that by a control of diesel tanks of vehicles, one will then not get the result until after the analysis is conducted in a laboratory. This is a considerable weakness with said method.

The present invention aims therefore to provide a method of detection which is simple to employ, and where the result is available immediately. The method according to the invention is based on detecting a dye by measurement of the light absorption in the diesel sample of interest.

US 5,002,397 discloses a system for fluid inspection and/or identification, whereas US 4,810,090 describes a method and means for monitoring concentration of components of blood flowing through a flowpath. US 5,696,592 disclose an apparatus for measuring light penetrability of liquids, and US 5,172,192 describes a spectrophotometric probe for in situ absorption spectra measurements. JP 60 044 851 relates to a spectrophotometer which is possible to immerse in a liquid sample.

The process according to the present invention for detecting dyes will however be independent of the degree of particle contamination in the sample, in that the absorption of light of the particles will be identical at the chosen light frequencies.

The present invention thus relates to a process for detecting in a fluid diesel sample dyed diesel in non-dyed diesel, there being transmitted through the fluid, via two light-emitting diodes, a first and a second beam of light of two different wavelengths, L1 and L2, and where the intensity of light of the two light beams which have propagated through the fluid is detected by two light-registering means, or one light-registering means if the two light beams are emitted sequentially, wherein the first light beam is emitted with a wavelength L1 of about 600 nm, and that the quantity of light of the first light beam which passes the sample is detected, and that the wavelength L2 of the second light beam is in the region of 380 - 450 nm, preferably about 410 nm, and that the quantity of light of the second light beam which passes the sample is detected, and that a decrease in the ratio of the detected light intensity of wavelength L1 to the detected light intensity of wavelength L2 is used as a measure of the presence of the dyed fluid in the non-dyed fluid.

Also described is a means, preferably designed as a measuring probe, for measuring fractions of dyed diesel in a sample of non-dyed dye, and/or for measuring the degree of particle contamination in a sample, said means comprising a cavity for reception of the fluid which is to be investigated, and where the arrangement on each side of the cavity is equipped with a number of light-emitting diodes and a number of light-detecting diode/s, where between the light-emitting diodes and the space there is arranged a first focussing lens, and that between the cavity and the light-detecting diode/s there is arranged a second focussing lens, characterised in that the first focussing lens is a planoconvex lens where the lens is arranged in the probe so that the convex surface of the lens is directed towards the light-emitting diode/s and where the plane surface of the lens is arranged adjacent to, or at a distance from, and parallel to a plane wall portion of the space, and where the second focussing lens is a planoconvex lens where the lens is arranged so that the convex surface of the lens faces towards the light-detecting diode/s and where the plane surface of the lens is arranged adjacent to, or at a distance from, and parallel to the plane wall portion of the space.

The present invention will now be described in more detail, with reference to the accompanying Figures, in which:
Fig. 1 shows a measuring probe according to the invention in a section in the longitudinal direction.
Fig. 2 shows the same measuring probe seen in a section at 90 degrees to the section of Figure 1.

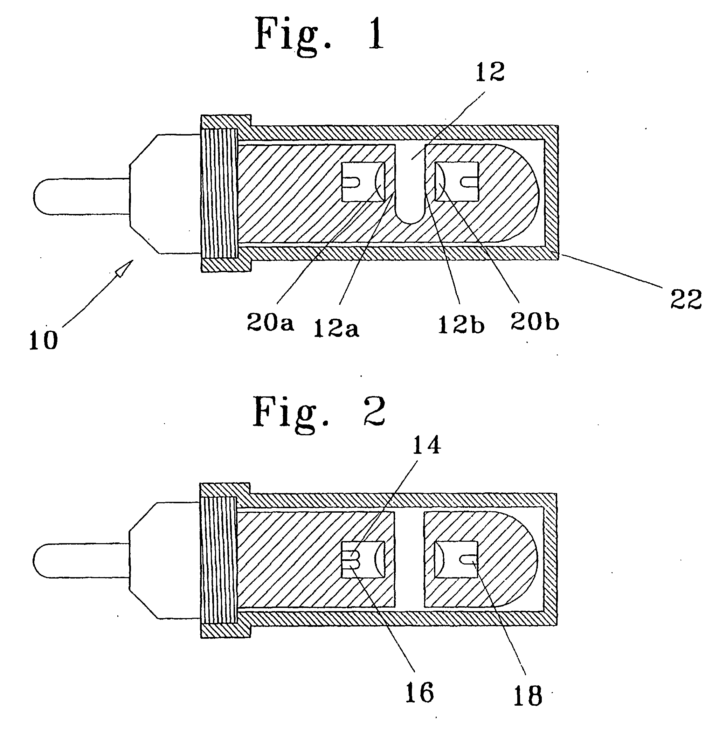

Fig. 1 shows an arrangement 10 . In the arrangement 10 there is arranged a cavity, i.e. a space 12, in which the actual fluid is received by lowering down the arrangement 10 in the fluid. The means 10 comprises two light-emitting diodes 14,16, which emit light of predetermined wave length, i.e. a light spectrum having a top of a given wavelength. Thus there is emitted light of two different wavelengths, L1 and L2. On the opposite side of the space 12, in relation to the emitting diodes 14,16, a number of light-detecting diodes 18 are arranged. If light is emitted simultaneously from the two emitting diodes 14,16 two detecting diodes 18 are required, while there is only a need for one detecting diode if the light is emitted sequentially from the two emitting diodes 14,16.

The means 10 comprises further in an embodiment two focusssing lenses (20a, 20b), and where these are preferably arranged cast into the probe itself so that all the light which is conducted by the lens (20a) strikes at right angles to the fluid in the space 12. This makes the measurements which are carried out independent of the refractive index of the fluid, and the measurements are therefore independent of which type of diesel oil the measurement is conducted on.

A cable connects the diodes 14,16,18 with a source of current and equipment for recording and detecting the light which has passed through the fluid. The detected light of the two wavelengths L1 and L2 are related to each other, and the value shows whether the diesel sample includes fractions of a diesel which is dyed.

The means 10 according to the invention is designed as a probe in a preferred embodiment. Thus it can be thrust down into a diesel sample, and the test result can be immediately read off on a display (not shown). The control authorities who conduct the test thus receive immediately a very reliable indication of whether the vehicle illegally employs the duty-free dyed diesel.

### Example 1

With a means 10 which is not equipped with focussing lenses there are conducted measurements on the same diesel distillate (base) of varying composition with respect to the mix ratio between dyed and non-dyed diesel. The results are given in Table I.

The accuracy of the measuring means is with respect to the second decimal in the ratio of detected light for L1 and L2, 600 nm and 430 nm, respectively. The results are accurate, and indicate with great reliability whether a diesel oil contains fractions of a dyed diesel. A mixture consisting of 97% non-dyed diesel and 3% dyed diesel yields measured results which are significantly different from a diesel sample which is not mixed with dyed diesel.

**Table I**

| **Sample** | **Transmission at 600 nm (relative units)** | **Transmission at 430 nm (relative units)** | **Transm. 600/ Transm. 430** |
|---|---|---|---|
| Calibration | 100 | 100 | |
| | | | |
| Duty-levied | 119,3 | 89,2 | 0,75 |
| non-dyed diesel | 119,2 | 88,7 | 0,75 |
| | 119,5 | 88,9 | 0,75 |
| 3% dyed diesel/ | 118,6 | 87,6 | 0,74 |
| 97 % undyed diesel | 118,7 | 87,9 | 0,74 |
| | 118,4 | 87,8 | 0,74 |
| 10% dyed diesel/ | 118,9 | 84,8 | 0,71 |
| 90% undyed diesel | 118,2 | 84,5 | 0,71 |
| | 118,9 | 84,8 | 0,71 |

It is evident from the results that even some few percentage parts of duty-free dyed diesel can be detected with reliability in a sample of undyed diesel.

The results are further independent of the degree of particle contamination in the sample, the absorption measurements for both the chosen wavelengths being influenced in the same proportion by particles in the solution.

### Example 2

The preceding Example clearly demonstrates the accuracy and the potential of the measuring method. However various diesel distillates exist on the market. Inter alia these different distillates include dissimilar amounts of aromatics, and these aromatics will give the different diesel samples different refractive indices, and the measured values will therefore deviate somewhat when focussing lenses are not employed. This is evident from Table II where the undyed diesel has a ratio between transmissions at 430 nm and 600 nm which varies from 0.77 - 0.90. However, as is evident from Table II, one can clearly determine qualitatively whether a diesel sample includes dye additives. Other analyses (for example DIN 51426) will thereafter confirm and quantify the dye additive.

The experimental tests which are conducted in the Examples 1 - 4 are made with a probe without focussing lenses.

**Table II**

| **Sample** | **Transmission at 600 nm (relative units)** | **Transmission at 430 nm (relative units)** | **Transm. 430/ Transm. 600** |
|---|---|---|---|
| | | | |
| Calibration | 100 | 100 | |

| **Undyed Diesel** | | | |
|---|---|---|---|
| Statoil | 120,2 | 94,4 | 0,79 |
| Shell | 123 | 94,4 | 0,77 |
| Esso | 131,4 | 96,1 | 0,77 |
| Hydro/Texaco | 119,7 | 97,7 | 0,82 |
| Fina | 121,5 | 109,1 | 0,90 |

| **Dyed Diesel** | | | |
|---|---|---|---|
| Statoil | 122,3 | 71,4 | 0,58 |
| Shell | 119,9 | 75,4 | 0,63 |
| Esso | 121,5 | 74 | 0,61 |
| Hydro/Texaco | 120,8 | 71,5 | 0,59 |
| Fina | 120,2 | 72,8 | 0,61 |

It is evident from the Table that the ratio is reduced for the duty-free dyed diesel as a consequence of the light passage of light having a wavelength of 430 nm being reduced.

### Example 3

Table III shows that the measuring method can either detect red dye which is added to the diesel or the tracer or a combination of these. The tracer is the chemical compound added so as to be able to conduct a chemical quantification. The dye is added first and foremost so as to be able to distinguish purely visually duty-free diesel from duty-levied diesel.

**Table III**

| **Sample** | **Transmission at 600 nm (relative units)** | **Transmission at 430 nm (relative units)** | **Transm. 430/ Transm. 600** |
|---|---|---|---|
| Calibration | 100 | 100 | |
| Sample with tracer | 121,5 | 64,4 | 0,53 |
| Sample with red dye | 117,8 | 56,9 | 0,48 |
| Sample red diesel | 115,9 | 50,2 | 0,43 |
| Undyed diesel | 122,2 | 94,6 | 0,77 |

Table IV shows an outline of which tracers and dyestuffs which are employed for the marking of mineral oils in Europe.

**Table IV**

| **OUTLINE OF EUROPEAN TRACERS FOR MARKING OF MINERAL OIL.** | | |
|---|---|---|
| Country | Dye | Tracer |
| Belgium | red dye | furfural |
| Denmark | none | none |
| Finland | red, same as in Norway | furfural |
| France | Solvent Red 24 | furfural+ diphenylamine |
| Greece (fuel oil) | Sudan Red | furfural |
| Irland (diesel) (paraffin) | Solvent Blue 79 Solvent Red 19 | Solvent Yellow 124 " |
| Italy (diesel, fishing boats) Light fuel oil | Alizarin Green G base Alizarin Green 3 B phase | furfural RS(2-ethyl-anthraquinone) Secret mix |
| Luxembourg | red dye | furfural |
| Netherlands diesel and light fuil oil | alkyl derivatives of azobenzene-4-azo-2-naphtol, (red dye) | furfural |
| Norway | Solvent Red 462 | Solvent Yellow 124 |
| Spain | red, same as in Norway, or a hydroxy-derivative of this | Solvent Yellow 124/ furfural |
| Great Britain (diesel) (paraffin) | Solvent Red 24 " | 1,4-dihydroxy anthraquinone coumarin |
| Sweden | Solvent Blue 79 | Solvent Yellow 124 |
| Germany | red dye * | furfural |

| | | |
|---|---|---|
| * the following red dyes are used in Germany, either separately or in mixture: 4-amino-azobenzene-2-ethylaminonaphthalene, 4-amino-azotoluene-2-tridecylaminonaphthalene or 4-amino-azotoluene-2-(2-ethyl)-hexylaminonaphthalene | | |

### Example 4

Table V shows the results of detection of 10% dyed diesel in undyed diesel

**Table V**

| **Sample** | **Transmission at 600 nm (relative units)** | **Transmission at 430 nm (relative units)** | **Transm. 430/ Transm. 600** |
|---|---|---|---|
| Calibration | 100 | 100 | |

| **Undyed diesel** | | | |
|---|---|---|---|
| Shell | 122,6 | 103 | 0,84 |
| Hydro/Texaco | 121,1 | 101,8 | 0,84 |
| Fina * | 121 | 108,5 | 0,90 |
| Esso | 116,3 | 98,8 | 0,85 |

| **Dyed diesel** | | | |
|---|---|---|---|
| Shell | 120,6 | 95, 9 | 0,79 |
| Hydro/Texaco | 120,1 | 95, 2 | 0,79 |
| Fina * | 119,7 | 104,8 | 0,87 |
| Esso | 114,8 | 92 | 0,80 |

As previously shown the measuring method according to the invention is sufficiently sensitive to be able to measure additives of only some few percent dyed diesel in undyed diesel. This is also evident from Table V where the relation proportions of each of the samples decreases with the addition of dye.

As mentioned, it is a problem that the base values for the different diesel samples have dissimilar transmission values, and also dissimilar proportions. Since we measure at two different wavelengths, as is indicated in claim 1, the degree of particles in the samples will not affect the result. However the arrangement which is employed for the Examples 1 - 4, i.e. without focussing lenses, will yield results which are dependent on the refractive index of the fluid. Different amounts of aromatics in the fluid will affect the refractive index, and hence the base values of the different diesel distillates. This explains that the sample from Fina yields high ratios.

By equipping the arrangement 10 with a set of focussing lenses 20a, 20b, which arrange for the light which is guided into the sample to strike the fluid at right angles, i.e. at right angles relative to the wall portions 12a and 12b of the cavity 12, i.e. as indicated in Fig. 1, the measuring results will be made independent of the refractive index of the fluid. The wall portions 12a, 12b of the cavity 12 are planar. With such a probe, as indicated in claim 7, one will expect that the base values, for dissimilar diesel distillates, i.e. in undyed samples, become more alike. When the base values are identical one will be able to determine fractions of dyed diesel of only a few per cent in an unknown diesel sample. Results as in Example 1 are expected.

Further, by the application of focussing lenses a significant increase in the sensitivity of the method is anticipated.

On measuring dye fractions it is not necessary to have a calibration of the measuring apparatus before use, since the results measured for the two different wavelengths are related to each other.

However the arrangement can also be employed for measuring fractions of particles in a solution if the apparatus is calibrated in advance in the dark, and where one thereafter measures the decline in the passage of light. There is a correlation between the quantity of particles in the solution and the reduction in transmission, and by establishing a standard curve for the transmission/ particle contamination ratio it will be possible to deduce from this how much a solution is with contaminated particles. By measurements at 600 nm this method will be independent of the amount of dye in the solution. This means that the means can simultaneously detect dye and particles respectively. By employing a set of focussing lenses the measurements will also be independent of refractive indies, and thus represents an improvement in relation to NO-177618.

In order to obtain a favorable calibration, the measuring probe is equipped with a case 22, constructed of light-proof material, so that calibration of the probe can be effected in the dark, if desired in the air or in a particle-free fluid. The case 22 can for example be fastened to the measuring probe by a set of threads 24.

## Claims

1. Process of detecting in a fluid diesel sample dyed diesel in non-dyed diesel, there being transmitted through the fluid, via two light-emitting diodes (14,16), a first and a second beam of light of two different wavelengths, L1 and L2, and where the intensity of light of the two light beams which have propagated through the fluid is detected by two light-registering means (18), or one light-registering means (18) if the two light beams are emitted sequentially, **characterised in that** the first light beam is emitted with a wavelength L1 of about 600 nm, and that the quantity of light of the first light beam which passes the sample is detected, and that the wavelength L2 of the second light beam is in the region of 380-450 nm, preferably about 410nm, and that the quantity of light of the second light beam which passes the sample is detected, and that a decrease in the ratio of the detected light intensity of wavelength L1 to the detected light intensity of wavelength L2 is used as a measure of the presence of the dyed fluid in the non-dyed fluid.

2. Process in accordance with claim 1, **characterized in that** the light beams from the emitting diodes (14,18) are guided via a first focusing lens (20a) perpendicularly to a plane wall portion (12a) of a fluid-receiving space (12), and where the light which has propagated through the fluid is guided out through a plane wall portion (12b) of the space (12), and thereafter through a second focusing lens (20b) to the detecting diode/s (18).

3. Process in accordance with one of the claims 1 - 2, **characterized in that** a decline in ratio of the transmission_{410 nm}/transmission_{600 nm} ratio relative to the ratio for an undyed sample, indicates that the sample includes fractions of dyed diesel.

## Patentansprüche

1. Verfahren zum Erfassen von gefärbtem Diesel in ungefärbtem Diesel in einer flüssigen Dieselprobe, wobei durch die Flüssigkeit über zwei Licht emittierende Dioden (14, 16) ein erster und ein zweiter Strahl von Licht mit zwei unterschiedlichen Wellenlängen L1 und L2, gesendet wird und wobei die Intensität des Lichtes der beiden Lichtstrahlen, die sich durch die Flüssigkeit ausgebreitet haben, durch zwei Licht anzeigende Einrichtungen (18) oder eine Licht anzeigende Einrichtung (18), wenn die beiden Lichtstrahlen nachfolgend emittiert werden, erfasst wird,
**dadurch gekennzeichnet,**
**dass** der erste Lichtstrahl mit einer Wellenlänge L1 von etwa 600 nm emittiert wird und dass die Lichtmenge des ersten Lichtstrahles, der die Probe passiert, erfasst wird und dass die Wellenlänge L2 des zweiten Lichtstrahles im Bereich von 380 - 450 nm, vorzugsweise etwa 410 nm, liegt und dass die Lichtmenge des zweiten Lichtstrahles, der die Probe passiert, erfasst wird und dass eine Verringerung des Verhältnisses der erfassten Lichtintensität der Wellenlänge L1 zu der erfassten Lichtintensität der Wellenlänge L2 als ein Maß des Vorhandenseins der gefärbten Flüssigkeit in der nicht gefärbten Flüssigkeit verwendet wird.

2. Verfahren nach Anspruch 1,
**dadurch gekennzeichnet,**
**dass** die Lichtstrahlen aus den emittierenden Dioden (14, 16) über eine erste fokussierende Linse (20a) senkrecht zu einem ebenen Wandbereich (12a) eines Flüssigkeit aufnehmenden Raumes (12) geführt werden, und wobei das Licht, das sich durch die Flüssigkeit ausgebreitet hat, durch einen ebenen Wandbereich (12b) des Raumes (12) und danach durch eine zweite fokussierende Linse (20b) zu der bzw. den detektierenden Diode(n) (18) geführt wird.

3. Verfahren nach einem der Ansprüche 1 - 2,
**dadurch gekennzeichnet,**
**dass** ein Senken des Verhältnisses des Transmission_{410 nm}/Transmission_{600 nm}-Verhältnisses relativ zu dem Verhältnis für eine ungefärbte Probe anzeigt, dass die Probe Fraktionen von gefärbtem Diesel aufweist.

## Revendications

1. Procédé de détection, dans un échantillon de diesel fluide, de diesel coloré dans du diesel non coloré, étant transmis à travers le fluide, via deux diodes électroluminescentes (14, 16), un premier et un second faisceaux lumineux de deux longueurs d'onde différentes, L1 et L2, et où l'intensité de lumière des deux faisceaux lumineux qui se sont propagés à travers le fluide est détectée par deux moyens d'enregistrement de lumière (18), ou un moyen d'enregistrement de lumière (18) si les deux faisceaux lumineux sont émis séquentiellement, **caractérisé en ce que** le premier faisceau lumineux est émis avec une longueur d'onde L1 d'environ 600 nm, et **en ce que** la quantité de lumière du premier faisceau lumineux qui traverse l'échantillon est détectée, et **en ce que** la longueur d'onde L2 du second faisceau lumineux se trouve dans la région de 380 à 450 nm, de préférence d'environ 410 nm, et **en ce que** la quantité de lumière du second faisceau lumineux qui traverse l'échantillon est détectée, et **en ce qu'**une diminution du rapport intensité de lumière détectée de longueur d'onde L1 sur intensité de lumière détectée de longueur d'onde L2 est utilisée comme mesure de la présence du fluide coloré dans le fluide non coloré.

2. Procédé selon la revendication 1, **caractérisé en ce que** les faisceaux lumineux provenant des diodes électroluminescentes (14, 16) sont guidés via une première lentille de mise au point (20a) perpendiculairement à une partie de paroi plane (12a) d'un espace de réception de fluide (12), et où la lumière qui s'est propagée à travers le fluide est guidée vers l'extérieur à travers une partie de paroi plane (12b) de l'espace (12), et ensuite par l'intermédiaire d'une seconde lentille de mise au point (20b) vers la(les) diode(s) de détection (18).

3. Procédé selon l'une des revendications 1 et 2, **caractérisé en ce qu'**un déclin du rapport transmission₄₁₀ₙₘ / transmission₆₀₀ₙₘ par rapport au rapport pour un échantillon non coloré, indique que l'échantillon comprend des fractions de diesel coloré.
